(19) Europäisches Patentamt
European Patent Office
Office européen des brevets

(11) **EP 4 410 311 A1**

(12) **EUROPEAN PATENT APPLICATION**
published in accordance with Art. 153(4) EPC

(43) Date of publication:
**07.08.2024 Bulletin 2024/32**

(21) Application number: **22876817.2**

(22) Date of filing: **28.09.2022**

(51) International Patent Classification (IPC):
*A61K 47/42* (2017.01)        *A61K 47/64* (2017.01)
*C07K 14/47* (2006.01)        *C07K 1/16* (2006.01)
*C12N 15/70* (2006.01)        *C07K 14/705* (2006.01)
*A61K 38/00* (2006.01)        *A61P 35/00* (2006.01)

(52) Cooperative Patent Classification (CPC):
**A61K 38/00; A61K 47/42; A61K 47/64;
A61P 35/00; C07K 1/16; C07K 14/47;
C07K 14/705; C12N 15/70**

(86) International application number:
**PCT/KR2022/014508**

(87) International publication number:
**WO 2023/055045 (06.04.2023 Gazette 2023/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.09.2021 KR 20210128551
07.04.2022 KR 20220043553
26.09.2022 KR 20220121834**

(71) Applicant: **Nbios, Inc.
Gangneung-si, Gangwon-do 25457 (KR)**

(72) Inventors:
• **LEE, Dae-Hee
Gangneung-si Gangwon-do 25516 (KR)**

• **LEE, Jeong Eun
Gangneung-si Gangwon-do 25494 (KR)**
• **LIM, Sung In
Busan 48050 (KR)**
• **SHIN, Goeun
Busan 46617 (KR)**
• **KWON, Hyuk Taek
Busan 48516 (KR)**
• **YANG, I Ji
Geoje-si Gyeongsangnam-do 53334 (KR)**

(74) Representative: **V.O.
P.O. Box 87930
2508 DH Den Haag (NL)**

Remarks:
The complete document including Reference
Table(s) and the Sequence Listing(s) can be
downloaded from the EPO website

(54) **COILED-COIL FUSION PROTEIN**

(57) The present disclosure relates to a polypeptide containing a self-assembling domain, a fusion protein containing the polypeptide, a nucleic acid molecule encoding the same, or a composition containing the polypeptide, fusion protein or nucleic acid molecule. The fusion protein or composition of the present disclosure can be combined or loaded with various drug moieties (proteins, antibody fragments, etc.) depending on desired purposes into a module form and can be combined through self-assembly to achieve improved binding to a target and/or increased drug activity.

FIG. 3A

→ w/o DTT

→ w/ DTT

**Description**

[Technical Field]

The present disclosure relates to a coiled-coil scaffold with improved binding stability due to co-expression. The coiled-coil scaffold presented in the present disclosure, wherein a drug moiety (protein, antibody fragment, etc.) is bound to a MBD2-p66α (methyl-binding domain protein 2-transcriptional repressor p66α) complex, is a fusion protein or a drug delivery vehicle that can implement complexes of various functional molecules into module forms.

[Background Art]

The drug delivery vehicles currently in use or under development are limited in the number and type of loaded drugs. In addition, the combination of the loaded drugs cannot be changed easily because the existing process of producing the drug delivery vehicles must be carried out in the same way.

[References of Related Art]

[Patent Documents]

US 2014-0073566 A1.

**[Disclosure]**

[Technical Problem]

The inventors of the present disclosure have made consistent efforts to develop a fusion protein or a platform of a drug delivery vehicle that allows easy loading of various types of drug moieties (therapeutic proteins, antibody fragments, etc.) and exhibits improved drug efficacy. As a result, they have identified that, when a drug moiety is bound to the N-terminal or C-terminal of a polypeptide containing positions 360-393 of the amino acid sequence of MBD2 and a polypeptide containing positions 138-178 of the amino acid sequence of p66α, the two polypeptides are bound through self-assembly and exhibit improved binding ability and/or drug activity to a target, and have completed the present disclosure.

The present disclosure is directed to providing a fusion protein which contains a polypeptide 1 containing a self-assembling domain 1 and a polypeptide 2 containing a self-assembling domain 2.

The present disclosure is also directed to providing a nucleic acid molecule encoding the fusion protein.

The present disclosure is also directed to providing a vector containing the nucleic acid molecule.

The present disclosure is also directed to providing an expression construct which contains a nucleic acid molecule 1 encoding the polypeptide 1 containing the self-assembling domain 1 and a nucleic acid molecule 2 encoding the polypeptide 2 containing the self-assembling domain 2.

The present disclosure is also directed to providing a host cell containing the vector or the expression construct or transformed with the vector or the expression construct.

The present disclosure is also directed to providing a pharmaceutical composition for preventing or treating cancer, which contains the fusion protein, nucleic acid molecule, vector, expression construct or host cell.

The present disclosure is also directed to providing a kit which contains the polypeptide 1 or the nucleic acid molecule 1 and the polypeptide 2 or the nucleic acid molecule 2.

Other purposes and advantages of the present disclosure become more apparent by the following detailed description, claims and drawings.

[Technical Solution]

In an aspect of the present disclosure, the present disclosure a fusion protein which contains a polypeptide 1 containing a self-assembling domain 1 and a polypeptide 2 containing a self-assembling domain 2.

In the present specification, the term "fusion protein" refers to a hybrid protein expressed by a nucleic acid molecule containing nucleotide sequences of at least two genes.

In the present specification, the term "self-assembling domain" refers to a protein that can self-assemble with another self-assembling domain to form a coiled coil. For example, the self-assembling domain 1 can self-assemble with the self-assembling domain 2 to form a coiled coil. The self-assembling domains can self-assemble via noncovalent bonding, i.e., hydrophobic and/or ionic interaction, between them to form a coiled coil.

The self-assembling domain 1 contains specifically an amino acid sequence of SEQ ID NO 1 or an amino acid sequence wherein an amino acid selected from a group consisting of the 6th and 27th amino acids of SEQ ID NO 1 is substituted with cysteine, more specifically an amino acid sequence wherein an amino acid selected from a group consisting of the 6th and 27th amino acids of SEQ ID NO 1 is substituted with cysteine.

The self-assembling domain 2 contains specifically an amino acid sequence of SEQ ID NO 6 or an amino acid sequence wherein an amino acid selected from a group consisting of the 8th and 29th amino acids of SEQ ID NO 6 is substituted with cysteine, more specifically an amino acid sequence wherein an amino acid selected from a group consisting of the 8th and 29th amino acids of SEQ ID NO 6 is substituted with cysteine.

The substitution with cysteine at the amino acid positions allows the formation of a more stable fusion protein by further inducing covalent bonding (disulfide bonding) between the two self-assembling domains.

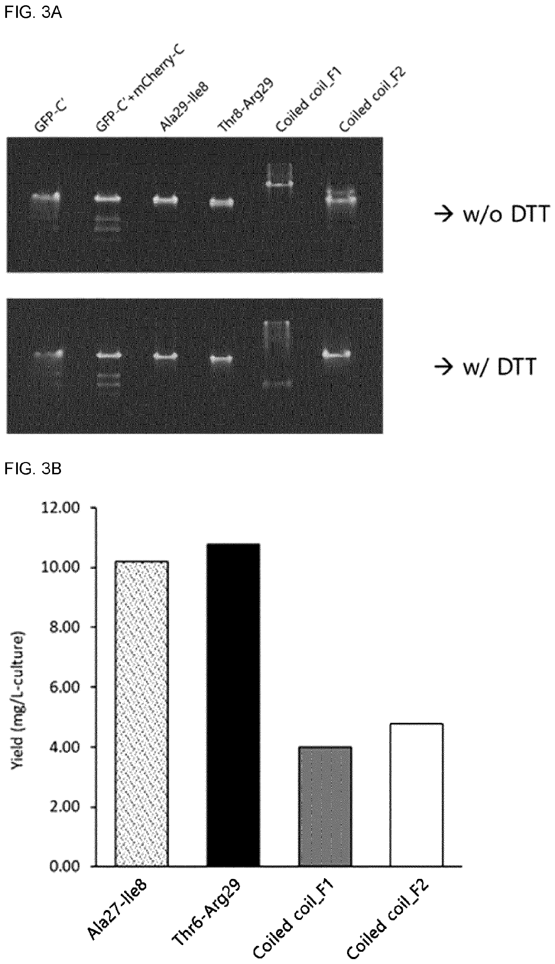

In an example of the present disclosure, it was confirmed that when the cysteine mutation is introduced at positions other than the cysteine mutation positions of the self-assembling domain 1 (the 6th and 27th positions in SEQ ID NO 1) or the cysteine mutation positions of the self-assembling domain 2 (the 8th and 29th positions in SEQ ID NO 6), e.g., at the 16th and 20th positions in SEQ ID NO 1 and/or the 18th and 11th positions in SEQ ID NO 6, a stable fusion protein is not formed or the expression level is decreased significantly (FIGS. 3a and 3b).

In a specific exemplary embodiment of the present disclosure, the self-assembling domain 1 contains an amino acid sequence of SEQ ID NO 2 or 3.

In a specific exemplary embodiment of the present disclosure, the self-assembling domain 2 contains an amino acid sequence of SEQ ID NO 7 or 8.

In a specific exemplary embodiment of the present disclosure, the fusion protein contains a self-assembling domain 1 containing an amino acid sequence of SEQ ID NO 2 and a self-assembling domain 2 containing an amino acid sequence of SEQ ID NO 7.

In a specific exemplary embodiment of the present disclosure, the fusion protein contains a self-assembling domain 1 containing an amino acid sequence of SEQ ID NO 3 and a self-assembling domain 2 containing an amino acid sequence of SEQ ID NO 8.

The fusion protein of the present disclosure may further contain one or more drug moiety.

In a specific exemplary embodiment of the present disclosure, the fusion protein contains a drug moiety at one or more site selected from a group consisting of the self-assembling domain 1 or the N-terminal and the C-terminal of the polypeptide 1 containing a self-assembling domain 1.

In a specific exemplary embodiment of the present disclosure, the fusion protein contains a drug moiety at one or more site selected from a group consisting of the self-assembling domain 2 or the N-terminal and the C-terminal of the polypeptide 2 containing a self-assembling domain 2.

The term "polypeptide 1" used in the present specification refers to the self-assembling domain 1 or a peptide containing the self-assembling domain 1, which has the property of self-assembling with the self-assembling domain 2 or a peptide containing the self-assembling domain 2.

The term "polypeptide 2" used in the present specification refers to the self-assembling domain 2 or a peptide containing the self-assembling domain 2, which has the property of self-assembling with the self-assembling domain 1 or a peptide containing the self-assembling domain 1.

The term "drug moiety" used in the present specification refers to a material that can interact with another protein or material such as an antibody or an antigen-binding fragment thereof or a protein (e.g., therapeutic protein, ligand, receptor or Fc region) or can conduct a specific function when expressed. The protein may be either a full-length protein or a domain or a part of a polypeptide that is responsible for a specific function, and may have a natural or artificial sequence.

In the present specification, the term "antibody" refers to protein molecule that acts as a receptor which specifically recognizes a specific antigen, including an immunoglobulin molecule having immunological activity for the antigen, and includes both polyclonal antibodies and monoclonal antibodies.

The antibody includes not only a complete full-length antibody but also the antigen-binding fragment (antibody fragment) of the antibody molecule. A complete antibody has a structure with two full-length light chains and two full-length heavy chains, wherein the light chains are connected to the heavy chains by disulfide bonds. The heavy-chain constant region has gamma ($\gamma$), mu ($\mu$), alpha ($\alpha$), delta ($\delta$) and epsilon ($\epsilon$) types and has gamma 1 ($\gamma$1), gamma 2 ($\gamma$2), gamma 3 ($\gamma$3), gamma 4 ($\gamma$4), alpha 1 ($\alpha$1) and alpha 2 ($\alpha$2) as subclasses. The light-chain constant region has kappa ($\kappa$) and lambda ($\lambda$) types.

In the present specification, the term "antigen-binding fragment of an antibody" refers to a fragment in the complete antibody molecule possessing the ability of recognizing binding specifically to an antigen and includes a single-domain antibody (sdAb), a single-chain antibody (scFv), Fab, F(ab'), F(ab')$_2$, Fv, etc. Examples of the fragment include a monovalent fragment (Fab fragment) consisting of $V_L$, $V_H$, $C_K$ (or $C_L$) and $C_H$1 domains; a bivalent fragment (F(ab')$_2$ fragment) containing two Fab fragments linked by a disulfide bridge at the hinge region; an Fd fragment consisting of $V_H$ and $C_H$1 domains; an Fv fragment consisting of the $V_L$ and $V_H$ domains of a single arm of an antibody and disulfide-linked Fv (sdFv); a dAb fragment consisting of a $V_H$ domain; and an isolated complementarity-determining region (CDR) or a combination of two or more isolated CDRs optionally linked by a linker. In addition, the $V_L$ and $V_H$ regions of scFv may be joined by a linker that enables them to be paired to form a single protein chain. These single-chain antibodies are also included in the antibody fragments. The antibody or antibody fragment includes a tetramer antibody containing two heavy-chain molecules and two light-chain molecules; an antibody light-chain monomer; an antibody heavy-chain monomer; an antibody light-chain dimer, an antibody heavy-chain dimer; an intrabody; a monovalent antibody; a camel antibody; and a single-domain antibody (sdAb).

In a specific exemplary embodiment of the present disclosure, the antigen-binding fragment of the antibody as the drug moiety is scFv.

In a specific exemplary embodiment of the present disclosure, the protein as the drug moiety is an albumin-binding peptide (ABP) or TRAIL (tumor necrosis factor-related apoptosis-inducing ligand).

The term "albumin-binding peptide" used in the present specification refers to a peptide or a protein binding to albumin which is a multifunctional plasma protein that plays an important role in maintaining osmotic pressure in blood, transportation of various substrates, etc. Because albumin has binding ability to FcRn, it has a long plasma half-life of 3 weeks. Therefore, the half-life can be increased by including the sequence of the albumin-binding peptide possessing binding activity to albumin in the fusion protein. Various types of albumin-binding peptides known in the art are disclosed in Zorzi et al., Medchemcomm. 2019 Jul 1; 10(7): 1068-1081, etc. which are incorporated in the present specification by reference.

The term "TRAIL" used in the present specification refers to a ligand that induces the apoptosis of tumor cells as one of tumor necrosis factors (TNFs). TRAIL has four receptors and, among these receptors, death receptors (DR4 and DR5) are overexpressed in cancer cells and decoy receptors (DcR1 and DcR2) are overexpressed in normal cells. The TRAIL can induce the death of tumor cells by binding to the death receptors such as DR4 and DR5 on the surface of the tumor cells. Because the decoy receptors do not have death domains at the C-terminal, the apoptotic signal is not transmitted into the cells. Therefore, the TRAIL-based therapy is a very effective next-generation anticancer therapy since toxicity is induced only in various cancer cells without harming normal cells. However, despite these advantages, TRAIL homotrimers, which are the active form of TRAIL monomers, have weak structural stability and in-vivo sustainability due to noncovalent linkage and, thus, are limited for clinical application.

In a specific exemplary embodiment of the present disclosure, the protein as the drug moiety is a TRAIL trimer.

The TRAIL trimer may form a trimer, hexamer, nonamer, dodeacemer, etc. in the fusion protein as it is bound to the N-terminal and/or C-terminal of the self-assembling domain 1 or the polypeptide 1 containing the self-assembling domain 1 or to the N-terminal and/or C-terminal of the self-assembling domain 1 or the polypeptide 2 containing the self-assembling domain 2.

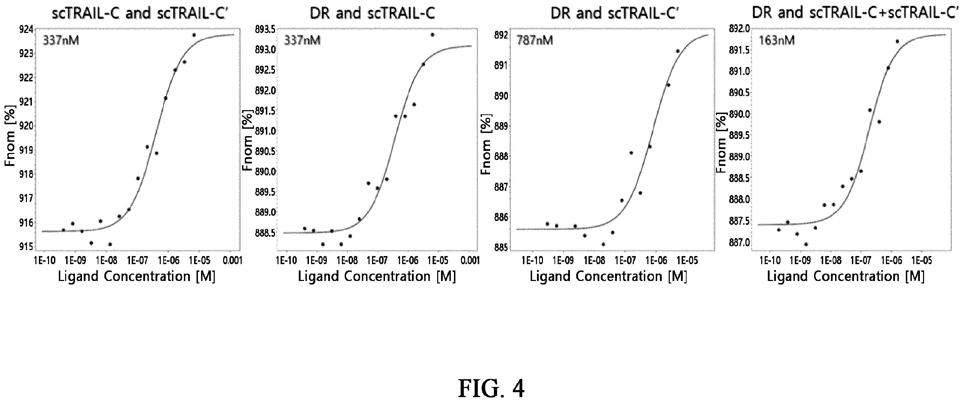

In an example of the present disclosure, it was confirmed that a TRAIL hexamer formed through self-assembly of TRAIL trimer-self-assembling domain 1 and TRAIL trimer-self-assembling domain 2 exhibits affinity to the death receptors increased by 2-5 times as compared to when 1) the TRAIL trimer-self-assembling domain 1 or 2) the TRAIL trimer-self-assembling domain 2 is expressed alone (FIG. 4).

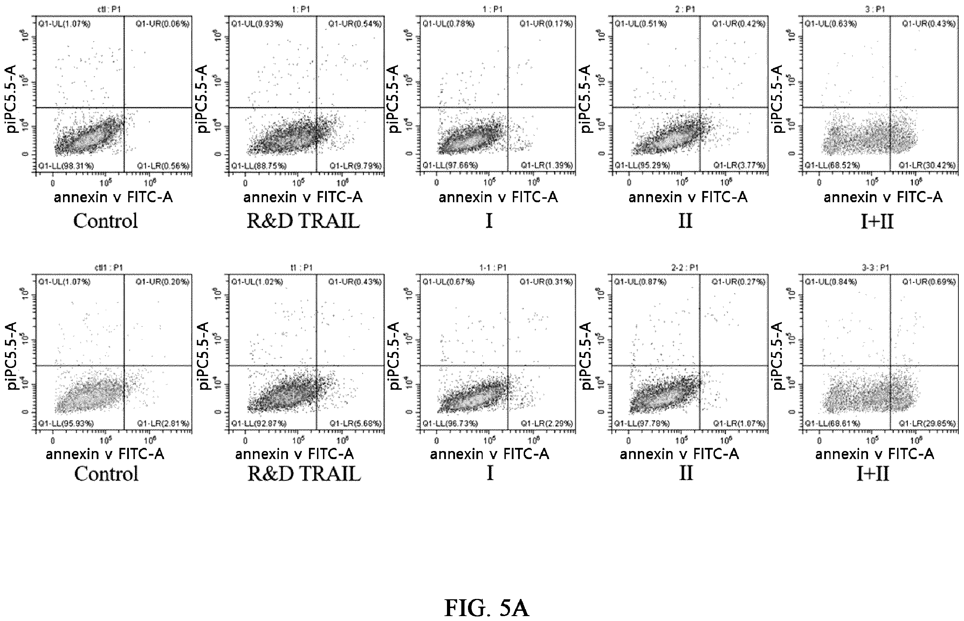

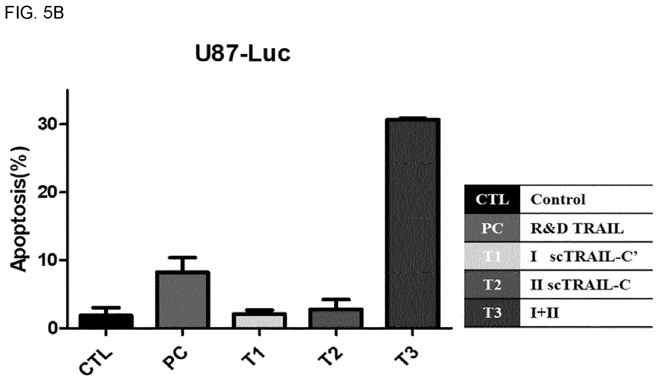

In another example of the present disclosure, it was confirmed that a TRAIL hexamer formed through self-assembly of TRAIL trimer-self-assembling domain 1 and TRAIL trimer-self-assembling domain 2 exhibits cancer cell death rate increased by 4 times or more as compared to when 1) the TRAIL trimer-self-assembling domain 1 or 2) the TRAIL trimer-self-assembling domain 2 is expressed alone (FIGS. 5a and 5b).

The terms "N-terminal" and "C-terminal" used in the present specification refer to the amino-terminal and carboxy-terminal of a polypeptide. A protein of the same kind (e.g., TRAIL trimer + TRAIL trimer, etc.) may be bound to the N-terminal and/or C-terminal of the self-assembling domain 1 or the polypeptide 1 containing the self-assembling domain 1 or the N-terminal and/or C-terminal of the self-assembling domain 2 or the polypeptide 2 containing the self-assembling domain 2, or a protein of different kinds (e.g., TRAIL trimer + ABP, TRAIL trimer + ABP + scFv, etc.) may be bound to the N-terminal and/or C-terminal. And, complexes of various functional molecules can be implemented into module forms.

In a specific exemplary embodiment of the present disclosure, the drug moiety may be bound to the N-terminal or C-terminal of the polypeptide 1 or 2 via a linker.

The term "linker" used in the present specification refers to an amino acid sequence that exists between the N-terminal or C-terminal of the polypeptide 1 or 2 and the N-terminal or C-terminal of the drug moiety. The linker may be any combination of amino acids, although not being limited thereto. A sequence may be introduced for the purpose of promoting the binding and dissociation of the polypeptide 1 or 2 and the drug moiety, providing flexibility for a coiled coil-forming domain, reducing steric hindrance that occurs during the binding of the coiled coil-forming domain, etc. Specifically, the linker may be a glycine- and serine-rich sequence of any length, e.g., Gly-Ser, Gly-Gly-Ser, Gly-Gly-Gly-Ser, Gly-Gly-Gly-Gly-Ser or a combination thereof, although not being limited thereto.

In a specific exemplary embodiment of the present disclosure, the present disclosure relates to a coiled-coil complex containing:

(i) a polypeptide 1 wherein an amino acid selected from a group consisting of the 6th and 27th amino acids of SEQ ID NO 1 and a combination thereof is substituted with cysteine; and

(ii) a polypeptide 2 wherein an amino acid selected from a group consisting of the 8th and 29th amino acids of SEQ ID NO 6 and a combination thereof is substituted with cysteine.

In another aspect of the present disclosure, the present disclosure provides a nucleic acid molecule encoding the fusion protein or the coiled-coil complex.

In the present specification, the term "nucleic acid molecule" comprehensively refers to DNA (gDNA and cDNA) and RNA molecules and includes not only natural nucleotides which are the basic constituents of nucleic acid molecules but also analogues with modified sugar or base moieties (Scheit, Nucleotide Analogs, John Wiley, New York (1980); Uhlman and Peyman, Chemical Reviews, (1990) 90: 543-584). The sequence of the nucleic acid molecule encoding the fusion protein of the present disclosure may be modified. The modification includes the addition, deletion or nonconservative or conservative substitution of the nucleotide.

The nucleic acid molecule of the present disclosure is interpreted to also include a nucleotide sequence exhibiting substantial identity to the nucleotide sequence described above. The substantial identity means that, when the nucleotide sequence of the present disclosure is aligned with another sequence to match as much as possible and the aligned sequences are analyzed using an algorithm commonly used in the art, they show at least 80% homology, at least 90% homology, or at least 95% homology.

In a specific exemplary embodiment of the present disclosure, the nucleotide sequence encoding the drug moiety may be contained at a site selected from a group consisting of the 5'- or 3'-end of the nucleic acid molecule encoding the polypeptide 1 (nucleic acid molecule 1) and/or the nucleic acid molecule encoding the polypeptide 2 (nucleic acid molecule 2).

In another aspect of the present disclosure, the present disclosure provides a vector containing the nucleic acid molecule.

In the present specification, the term "vector" refers to a delivery vehicle into which a polynucleotide sequence can be inserted for introduction into a cell capable of replicating the nucleic acid molecule sequence. The polynucleotide sequence may be exogenous or heterologous. The vector may be a plasmid, a cosmid vector or a viral vector (retroviral, adenoviral or adeno-associated viral vector), although not being limited thereto. Those skilled in the art can construct the vector according to standard recombinant techniques (Maniatis, et al., Molecular Cloning, A Laboratory Manual, Cold Spring Harbor Press, Cold Spring Harbor, N.Y., 1988; Ausubel et al., In: Current Protocols in Molecular Biology, John, Wiley & Sons, Inc., NY, 1994).

In the present specification, the term "expression vector" refers to a vector containing a nucleotide sequence encoding at least a part of a gene product to be transcribed. In some cases, an RNA molecule is then translated into a protein, a polypeptide or a peptide. The expression vector may contain various regulatory sequences. In addition to regulatory sequences that regulate transcription and translation, nucleic acid sequences that provide other functions may also be contained in the expression vector. The expression vector may contain the nucleic acid molecule 1 encoding the polypeptide 1 and the nucleic acid molecule 2 encoding the polypeptide 2 either in the same vector or in different vectors. The nucleic acid molecules 1 and 2 can form a fusion protein as the polypeptides 1 and 2 expressed from the same or different vectors are bound through self-assembly either in vitro or in vivo.

In another aspect of the present disclosure, the present disclosure provides a host cell which contains the vector or the expression construct or is transformed with the vector or the expression construct.

In the present specification, the term "cell" includes eukaryotic and prokaryotic cells and refers to any transformable cell which can replicate the vector or can express a gene encoded by the vector. The cell can be transfected, transduced or transformed by the vector, which refers to the process in which an exogenous polynucleotide (nucleic acid molecule) is transferred or introduced into the host cell. In the present specification, the term "transformation" is used to include transfection and transduction.

The (host) cell of the present disclosure may be, specifically an insect cell or a mammalian cell, more specifically an insect cell such as Sf9 or a mammalian cell such as HEK293 cell, HeLa cell, ARPE-19 cell, RPE-1 cell, HepG2 cell, Hep3B cell, Huh-7 cell, C8D1a cell, Neuro2A cell, CHO cell, MES13 cell, BHK-21 cell, COS7 cell, COP5 cell, A549 cell, MCF-7 cell, HC70 cell, HCC1428 cell, BT-549 cell,PC3 cell, LNCaP cell, Capan-1 cell, Panc-1 cell, MIA PaCa-2 cell, SW480 cell, HCT166 cell, LoVo cell, A172 cell, MKN-45 cell, MKN-74 cell, Kato-III cell, NCI-N87 cell, HT-144 cell, SK-MEL-2 cell, SH-SY5Y cell, C6 cell, HT-22 cell, PC-12 cell or NIH3T3 cell, although not being limited thereto.

Specifically, the host cell of the present disclosure is an isolated host cell.

In another aspect of the present disclosure, the present disclosure provides a composition containing the fusion protein, the nucleic acid molecule, the vector, the expression construct or the cell.

In another aspect of the present disclosure, the present disclosure provides a composition containing i) a polypeptide 1 containing an amino acid sequence of SEQ ID NO 2 or 3 or a nucleic acid molecule 1 encoding the polypeptide 1 as a self-assembling domain 1 and ii) a polypeptide 2 containing an amino acid sequence of SEQ ID NO 7 or 8 or a nucleic acid molecule 2 encoding the polypeptide 2 as a self-assembling domain 2.

The description of the "polypeptide", "nucleic acid molecule", "drug moiety", etc. will be omitted to avoid complexity and redundancy.

A drug moiety may be bound to one or more site selected from a group consisting of the N-terminal and C-terminal of the polypeptide 1 containing the amino acid sequence of SEQ ID NO 2 or 3 and the polypeptide 2 containing the amino acid sequence of SEQ ID NO 7 or 8 contained in the composition.

Accordingly, the composition may contain i) polypeptide 1-drug moiety, drug moiety-polypeptide 1 or drug moiety-polypeptide 1-drug moiety and ii) polypeptide 2-drug moiety, drug moiety-polypeptide 2 or drug moiety-polypeptide 2-drug moiety.

In addition, a nucleotide sequence encoding a drug moiety may be bound to a site selected from a group consisting of the 5'- or 3'-end of the nucleic acid molecule 1 encoding the polypeptide 1 containing the amino acid sequence of SEQ ID NO 2 or 3 and the nucleic acid molecule 2 encoding the polypeptide 2 containing the amino acid sequence of SEQ ID NO 7 or 8 contained in the composition.

The nucleic acid molecule 1, the nucleic acid molecule 2 and the nucleotide sequence encoding the drug moiety bound thereto may exist in the composition as being inserted in one or more vector.

In a specific exemplary embodiment of the present disclosure, the composition is a pharmaceutical composition for preventing or treating cancer.

In another aspect of the present disclosure, the present disclosure provides a method for preventing or treating cancer, which includes a step of administering a pharmaceutically effective amount of the fusion protein, the nucleic acid molecule, the vector, the expression construct, the cell or the composition to a subject.

In another aspect of the present disclosure, the present disclosure provides a use of the fusion protein, the nucleic acid molecule, the vector, the expression construct, the cell or the composition in therapy.

In a specific exemplary embodiment of the present disclosure, the use in therapy is a use for treating cancer.

In the present specification, the term "prevention" refers to suppressing a disease or the onset of the disease in a subject who has not been diagnosed as having the disease but is likely to develop the disease.

In the present specification, the term "treatment" refers to (a) suppression of the development of a disease or symptoms; (b) alleviation of a disease or symptoms; or (c) elimination of a disease or symptoms.

Therefore, the composition of the present disclosure may be used as a composition for treating cancer as it is or may be used as a therapeutic adjuvant that improves treatment responsiveness together with another pharmacological ingredient. Accordingly, in the present specification, the term "treatment" or "therapeutic agent" includes the meaning of "therapeutic acid" or "therapeutic adjuvant".

In the present specification, the term "administration" or "administer" refers to administering a therapeutically effective amount of the composition of the present disclosure directly to a subject so that the same amount is formed in the subject's body.

In the present disclosure, the term "therapeutically effective amount" means the content of the pharmaceutical composition of the present disclosure that contains the pharmacological ingredient in an amount sufficient to provide a therapeutic or prophylactic effect. Therefore, it includes the meaning of "prophylactically effective amount".

In the present specification, the term "subject" includes human, mouse, rat, guinea pig, dog, cat, horse, cow, pig, monkey, chimpanzee, baboon or rhesus monkey, without limitation. Specifically, the subject of the present disclosure is human.

Pharmaceutically acceptable carriers, excipients or stabilizers as those described in Remington's Pharmaceutical Sciences (1990) Mack Publishing Co., Easton, PA may be used in the composition of the present disclosure. The acceptable carriers, excipients or stabilizers are nontoxic to recipients at the administered dosages and concentrations and include buffers, e.g., phosphate, citrate and other organic acids; antioxidants including ascorbic acid and methionine; preservatives (e.g., octadecyl dimethyl benzyl ammonium chloride; hexamethonium chloride; benzalkonium chloride; phenol, butyl or benzyl alcohol; alkyl parabens, e.g., methyl or propyl paraben; catechol; resorcinol; cyclohexanol; 3-pentanol; and m-cresol); low-molecular-weight (less than about 10 residues) polypeptides; proteins, e.g., serum albumin, gelatin or immunoglobulin; hydrophilic polymers, e.g., polyvinylpyrrolidone; amino acids, e.g., glycine, glutamine, asparagine, histidine, arginine or lysine; monosaccharides such as glucose or mannose and other carbohydrates including disaccharides or dextrin; chelating agents, e.g., EDTA; sugars, e.g., sucrose, mannitol, trehalose or sorbitol; salt-forming counterions, e.g., sodium; metal complexes (e.g., Zn-protein complexes); and/or nonionic surfactants, e.g., Tween®, Pluronics® or polyethylene glycol (PEG).

In some exemplary embodiments, the pharmaceutical composition may contain the fusion protein, nucleic acid molecule, vector, expression construct or cell disclosed in the present specification in a pharmaceutically acceptable carrier. In specific exemplary embodiments, the pharmaceutical composition contains an effective amount of the fusion protein, nucleic acid molecule, vector, expression construct or cell disclosed in the present specification and, optionally, one or more additional prophylactic or therapeutic agent in a pharmaceutically acceptable carrier. In some exemplary embodiments, the fusion protein, nucleic acid molecule, vector, expression construct or cell is the only active ingredient contained in the pharmaceutical composition.

Pharmaceutically acceptable carriers used in parenteral preparations include aqueous vehicles, nonaqueous vehicles, antibacterial agents, isotonic agents, buffers, antioxidants, local anesthetics, suspending agents, dispersants, emulsifiers, metal ion sequestrants, chelating agents and other pharmaceutically acceptable materials. Examples of the aqueous vehicles include sodium chloride solution, Ringer's solution, isotonic dextrose solution, sterilized water for injection, Ringer's dextrose solution and Ringer's lactate solution. The nonaqueous vehicles include plant-derived fixed oils, cottonseed oil, corn oil, sesame oil and peanut oil. Antibacterial agents of bacteriostatic or fungicidal concentrations may be added to a parenteral administration packaged in a multi-dose container and they include phenol, cresol, mercurycontaining substances, benzyl alcohol, chlorobutanol, methyl and propyl p-hydroxybenzoate ester, thimerosal, benzalkonium chloride and benzethonium chloride. The isotonic agents include sodium chloride and dextrose. The buffers include phosphate and citrate. The antioxidants include sodium bisulfate. The local anesthetics include procaine hydrochloride. The suspending agents or dispersants include sodium carboxymethyl cellulose, hydroxypropylmethyl cellulose and polyvinylpyrrolidone. The emulsifiers include polysorbate 80 (Tween® 80). The metal ion sequestrant or, chelating agents include EDTA. The pharmaceutical carriers include ethyl alcohol, polyethylene glycol and propylene glycol for watermiscible vehicles and sodium hydroxide, hydrochloric acid, citric acid or lactic acid for pH adjustment.

The pharmaceutical composition may be formulated to suit the administration route for a subject. Specific examples of the administration route include intranasal, oral, intraspinal, intracerebroventricular, pulmonary, subcutaneous or intrathecal routes. Parenteral administration by subcutaneous, intramuscular or intravenous injection is also considered. For injection, the pharmaceutical composition may be prepared into a liquid solution, a suspension, a solid formulation suitable to be prepared into a solution or a suspension before injection, or an emulsion. The injectable solution or emulsion also contains one or more excipient. Suitable excipients include, for example, water, saline, dextrose, glycerol or ethanol. In addition, if desired, the pharmaceutical composition may contain small amounts of nontoxic adjuvants, e.g., a wetting agent, an emulsifier, a pH buffer, a stabilizer, a solubility enhancer, and other agents, e.g., sodium acetate, sorbitan monolaurate, triethanolamine oleate and cyclodextrin.

Preparations for parenteral administration of the pharmaceutical composition include sterile solutions that can be injected readily, sterile dry soluble products that can be combined with a solvent immediately before use, e.g., a tablet for subcutaneous injection or a lyophilized powder, sterile suspensions that can be injected readily, sterile dry insoluble products that can be combined with a vehicle immediately before use, and sterile emulsions. The solution may be aqueous or nonaqueous.

The pharmaceutical composition disclosed in the present specification may be prepared to target a specific tissue to be treated, a receptor or other body parts. Various targeting methods are known to those skilled in the art. Use of these targeting methods may be considered for the composition of the present disclosure. Non-limiting examples of the targeting methods include those described in US Patent Nos. 6,316,652, 6,274,552, 6,271,359, 6,253,872, 6,139,865, 6,131,570, 6,120,751, 6,071,495, 6,060,082, 6,048,736, 6,039,975, 6,004,534, 5,985,307, 5,972,366, 5,900,252, 5,840,674, 5,759,542 and 5,709,874. In specific exemplary embodiments, the pharmaceutical composition disclosed in the present specification may be used for treating cancer.

The composition may be sterilized for administration into the body. For example, it may be sterilized by filtration through a sterile filtration membrane.

In another aspect of the present disclosure, the present disclosure provides a kit containing:

(i) a polypeptide 1 wherein an amino acid selected from a group consisting of the 6th and 27th amino acids of SEQ ID NO 1 and a combination thereof is substituted with cysteine or a nucleic acid molecule 1 encoding the same;
(ii) a polypeptide 2 wherein an amino acid selected from a group consisting of the 8th and 29th amino acids of SEQ ID NO 6 and a combination thereof is substituted with cysteine or a nucleic acid molecule 2 encoding the same; and
(iii) a nucleotide sequence encoding a drug moiety bound to a site selected from a group consisting of the N-terminal and C-terminal of the polypeptides 1 and 2 or the 5'- or 3'-end of the nucleic acid molecules 1 and 2.

The description of the "polypeptide", "nucleic acid molecule", "drug moiety", etc. will be omitted to avoid complexity and redundancy.

In some exemplary embodiments, the present specification discloses a pharmaceutical pack or kit containing: one or more container filled with (i) the polypeptide 1 or the nucleic acid molecule 1 encoding the same, (ii) the polypeptide 2 or the nucleic acid molecule 2 encoding the same and (iii) a nucleotide sequence encoding a drug moiety bound to a site selected from a group consisting of the N-terminal and C-terminal of the polypeptides 1 and 2 or the 5'- or 3'-end of the nucleic acid molecules 1 and 2; and, optionally, an instruction for use. In some exemplary embodiments, the kit contains the pharmaceutical composition disclosed in the present specification and any prophylactic or therapeutic agent disclosed in the present specification. In some exemplary embodiments, the kit contains an instruction about the composition disclosed in the present specification.

[Advantageous Effects]

The features and advantages of the present disclosure may be summarized as follows:

(i) The present disclosure provides a polypeptide containing a self-assembling domain, a fusion protein containing the polypeptide or a nucleic acid molecule encoding the same, and a composition containing the polypeptide, fusion protein or nucleic acid molecule.

(ii) The fusion protein of the present disclosure can be implemented into a module form by binding or loading various drug moieties (proteins, antibody fragments, etc.) depending on desired purposes and can achieve improved binding ability and/or drug activity for the target by binding through self-assembly.

[Brief Description of Drawings]

FIG. 1a shows a result of predicting the structure of coil C'-GFP-6xHis and coil C-mCherry-6xHis using Alphafold2.

FIG. 1b shows a result of measuring the molecular weight of purified C'-GFP and C-mCherry by SDS-PAGE.

FIG. 2a shows a result of conducting native PAGE in order to confirm whether coil-fused fluorescent proteins were folded properly.

FIG. 2b shows a result of determining binding parameters by investigating the thermodynamics between C'-GFP and C-mCherry by isothermal titration calorimetry (ITC).

FIG. 3a shows a result of confirming whether Ala27-Ile8, Thr6-Arg29, Coiled coil_F1 and Coiled coil_F2 form stable complexes.

FIG. 3b shows a result of comparing the relative expression levels of Ala27-Ile8, Thr6-Arg29, Coiled coil_F1 and Coiled coil_F2.

FIG. 3c shows a result of comparing the FRET efficiency of Thr6-Arg29 and C'-GFP/C-mCherry with the FRET efficiency of Ala27-Ile8 as 100%.

FIG. 4 shows a result of comparing the affinity between TRAIL hexamer and a death receptor (DR and scTRAIL-C + scTRAIL-C') with the affinity between TRAIL trimer-C or TRAIL trimer- C' and the death receptor (DR and scTRAIL-C or DR and scTRAIL-C').

FIG. 5a shows a result of observing glioblastoma cells treated with a positive control group (R&D TRAIL), a TRAIL trimer-C (I) or TRAIL trimer-C'(II) test group and a TRAIL hexamer test group (I + II) by flow cytometry (FACS).

FIG. 5b shows a result of comparing the cancer cell death rate of a positive control group (PC), a TRAIL trimer-C (T1) or TRAIL trimer-C'(T2) test group and a TRAIL hexamer test group (T3).

[Best Mode]

Hereinafter, the present disclosure will be described in more detail through examples. The examples are provided only to describe the present disclosure more specifically and it will be obvious to those having ordinary knowledge in the art that the scope of the present disclosure is not limited by the examples.

**Examples**

**Materials and methods**

**1. Materials**

NaCl (sodium chloride), imidazole, NaOH (sodium hydroxide), tris(hydroxymethyl)aminomethane, glycerol (99.8%), methyl alcohol (99.8%), acetic acid (99.7%) and $H_2PO_4$ (dihydrogen phosphate) were purchased from Samchun. Yeast extract, agar, 6 N HCl (hydrochloric acid) and bromophenol blue were purchased from Daejung. BL21 (DE3) chemically competent *E.coli* was purchased from Enzynomics. 10% SDS solution, DTT (D/L-dithiothreitol) and Coomassie brilliant blue G-250 stain solution were purchased from Biosesang. Bacto tryptone was purchased from BD DIFCO. Ampicillin sodium and lysozyme were purchased from Sigma. Isopropyl β-D-1-thiogalactopyranoside (IPTG) was purchased from Bioneer. DPBS (Dulbecco's phosphate-buffered saline) was purchased from Welgene. Glycine was purchased from Duksan. Tris/glycine SDS pre-cast gel 8-16% and tris/glycine non-SDS pre-cast gel 12% were purchased from Koma Biotech. A polypropylene column and a PD10 column were purchased respectively from Qiagen and GE Healthcare. Unstained protein MW markers from Pierce Biotechnology Inc. were used for SDS-PAGE analysis.

## 2. Establishment of plasmids and bacterial strains

An insert encoding the coiled-coil region of human MBD2 or mutants thereof (amino acids 360-393, SEQ ID NOS 1-5) or p66 or mutants thereof (amino acids 138-178, SEQ ID NOS 6-10) was inserted into pET21a having resistance to ampicillin. pET-GFP-p66α was produced by cloning GFP-p66α between Ndel and Xhol of the pET21a and mCherry-MBD2 was also cloned between Ndel and Xhol of the pET21a. The experiment was conducted by Bionics.

[Table 1]

| Peptide | Mutation | SEQ ID NO | Sequence |
|---------|----------|-----------|----------|
| MBD2 | Wild type | 1 | KAFIVTDEDIRKQEERVQQVRKKLEEALMADILS |
| | A27C | 2 | KAFIVTDEDIRKQEERVQQVRKKLEECLMADILS |
| | T6C | 3 | KAFIVCDEDIRKQEERVQQVRKKLEEALMADILS |
| | R16C | 4 | KAFIVTDEDIRKQEECVQQVRKKLEEALMADILS |
| | V20C | 5 | KAFIVTDEDIRKQEERVQQCRKKLEEALMADILS |
| p66α | Wild type | 6 | PEERERMIKQLKEELRLEEAKLVLLKKLRQSQIQ KEATAQK |
| | I8C | 7 | PEERERM**C**KQLKEELRLEEAKLVLLKKLRQSQI QKEATAQK |
| | R29C | 8 | PEERERMIKQLKEELRLEEAKLVLLKKL**C**QSQIQ KEATAQK |
| | E18C | 9 | PEERERMIKQLKEELRL**C**EAKLVLLKKLRQSQIQ KEATAQK |
| | L11C | 10 | PEERERMIKQ**C**KEELRLEEAKLVLLKKLRQSQIQ KEATAQK |
| Sequences of human MBD2 or mutants thereof and p66α or mutants thereof | | | |

## 3. Protein expression and purification

### - Culturing

After thawing BL21 [DE3] (chemically competent cells) on ice, 50 μL was placed in a microcentrifuge tube and 1 μL of an expression vector was added. For negative control, two microcentrifuge tubes were prepared, one with the expression vector added and the other not. The tubes were placed on ice for 30 minutes and heat shock was applied at 42 °C for 30 seconds. After placing on ice for 2 minutes and then adding 200 μL of Luria-Bertani medium, 70 μL was taken from each tube and absorbed onto a Luria-Bertani agar plate. Finally, colonies sampled for about 12 hours or longer in an incubator set to 37 °C were added to 5 mL of Luria-Bertani medium and incubated overnight at 37 °C and 200 rpm in a shaking incubator. 400 mL of the Luria-Bertani medium was transferred to a 1-L Erlenmeyer flask and placed in a shaking incubator set to 37 °C and 200 rpm. After adding 400 μL of ampicillin and adding the culture from each tube, expression was induced with isopropyl β-D-1-thiogalactopyranoside at $A_{600}$ of 0.6. The cells were collected when the $A_{600}$ reached 2. The absorbance was measured using the Biodrop Duo micro-volume spectrophotometer (Biodrop).

**- Cell harvesting/lysis**

The medium was divided exactly in half, placed in Oak Ridge centrifuge tubes and centrifuged at 4000 rcf for 10 minutes at 4 °C. After discarding the supernatant, the precipitated cells were stored at -20 °C. The cell pellets were lysed by adding 20 mL of a lysis buffer. Then, the resulting solution was transferred to a 50-mL conical tube. After adding 400 µL of lysozyme and mixing at 4 °C for 30 minutes using a rotator, the mixed solution was sonicated on ice. After sonication for 7 minutes, followed by centrifugation at 10000 rcf for 20 minutes at 4 °C, the precipitates were removed and the supernatant was collected.

**- Affinity purification**

After adding 1.5 mL of Ni-NTA agarose to the solution, followed by mixing with a rotator for 10 minutes, the solution was added to a polypropylene column (5 mL) to obtain proteins only from the solution. About 15 mL of a wash buffer was flown onto beads to remove impurities. After adding 500 µL of an elution buffer to the beads, 5 times per each, the solution discharged from the column was added to a sterilized microtube. To obtain purified GFP-p66alpha and mCherry-MBD2 from the elution buffer, the buffer was replaced with PBS. After filling the PD10 column with PBS, 2.5 mL of the protein-containing solution was added and 3 mL of PBS was added.

**- Size-exclusion chromatography (FPLC)**

Size-exclusion chromatography (SEC) was conducted using a PBS-equilibrated ProteoSEC Dynamic 11/30 3-70 HR SEC column (Protein Ark, UK). The sample eluted from the Ni-NTA was concentrated with a centrifugal filter, filtered with a 0.22-µm syringe filter and loaded in a column. After conducting chromatography on AKTAprime plus using PBS at a flow rate of 1 mL/min, the eluted protein fractions were collected separately.

**4. Protein electrophoresis**

The samples collected from the various stages of protein purification were analyzed on 12% discontinuous polyacrylamide gel containing sodium dodecyl sulfate (SDS). The protein sample was mixed with pure distilled water and incubated at 98 °C for 7 minutes before separation by SDS-PAGE. The gel was stained with Coomassie brilliant blue to visualize proteins. Native PAGE was conducted on 12% polyacrylamide gel for 2 hours at the maximum voltage of 125 V. Then, the fluorescence of proteins was observed by irradiating UV.

**5. Isothermal titration calorimetry**

ITC analysis was conducted using MicroCal Auto-iTC200 (Malvern Panalytical) at the Korea Basic Science Institute (Ochang, Korea). Binding affinity was measured in a PBS buffer (pH 7.5) using 40 µL of C'-GFP and 200 µl of C-mCherry. For titration, 2 µl of C'-GFP was injected to C-mCherry 19 times at 25 °C for 4 seconds, with 150-seconds intervals, and data were analyzed using the MicroCal Origin 7.0 software. N is the stoichiometric number, $K_D$ is the binding affinity, and $\Delta H$ and $\Delta S$ are changes in enthalpy and binding entropy, respectively.

**6. Size-exclusion chromatography (HPLC)**

For analysis of protein-protein interaction, a Cytiva superdex 200 increase 10/300 GL column equilibrated with PBS (pH 7.5) was used in the YL HPLC system (Semi-prep) (YL9100S HPLC). The samples (C'-GFP and C-mCherry or C'-GFP/C-mCherry) were loaded in the column either alone or after being mixed at the equimolar ratio. The eluent was monitored by detecting absorbance at 280 nm.

**7. Fluorescence resonance energy transfer (FRET) analysis**

A sample of wild-type GFP alone or a mixture of C'-GFP/C-mCherry, Ala27-Ile8 and Thr6-Arg29 in equimolar quantities was diluted appropriately with phosphate-buffered saline (PBS) to make a total volume of 200 µL and a concentration of 4 µM. The sample was transferred to a 96-well plate and the absorbance at 510 nm 488 nm and luminescence (fluorescence) of each well were measured with the Synergy H1 hybrid multi-mode microplate reader (BioTek). After calculating the luminescence (A) per unit absorbance of the wild-type GFP alone sample and the luminescence (B) per unit absorbance of each sample, fluorescence resonance energy transfer efficiency was calculated by the following equation.

$$\text{Fluorescence resonance energy transfer (FRET) efficiency (\%)} = 100 \times \left(1 - \frac{B}{A}\right)$$

**Experimental results**

**1. Production and characterization of fluorescent protein-fused wild-type coil**

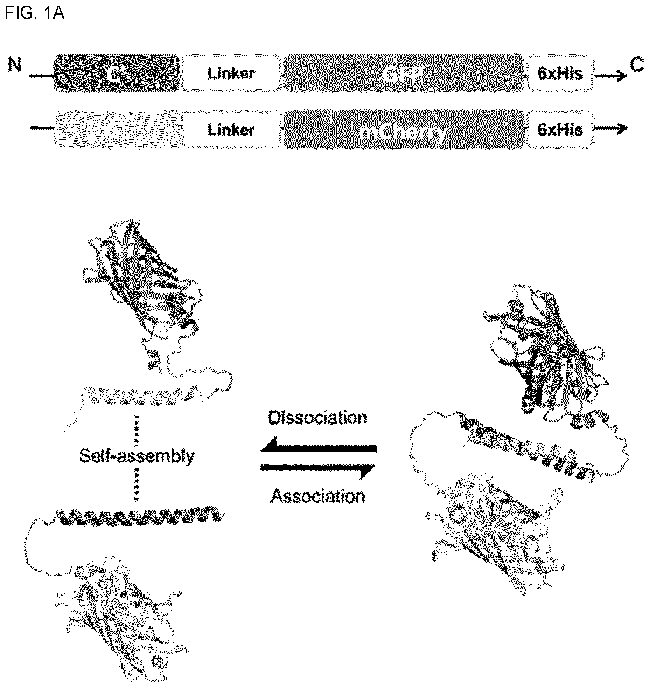

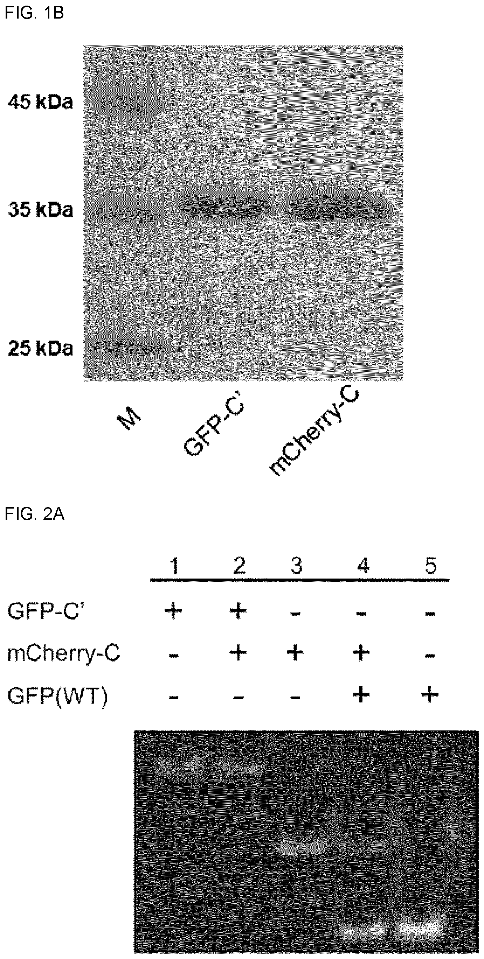

In order to prepare a non-natural heterodimeric protein complex using a coiled coil as a scaffold, the coiled-coil regions of p66alpha and MBD2, i.e., amino acids 138-178 (coil C') and 360-393 (coil C), that self-assemble complementarily to each other were used. The green fluorescent protein (GFP) and mCherry, which is one of red fluorescent proteins, were used to visually track the expression of the coil and complex formation. The GFP and mCherry were fused to the C-terminal of the coil C' and coil C, respectively. The 6-histidine (6xHis) tag was additionally fused to the C-terminal of each fluorescent protein and the protein was purified using nickel-nitrilotriacetic acid agarose resin. The complicated structures of the coil C'-GFP-6xHis fusion protein (hereinafter, referred to as C'-GFP) and the coil C-mCherry-6xHis fusion protein (hereinafter, referred to as C-mCherry) were predicted using Alphafold2 (FIG. 1a). The pET-C'-GFP and pET-C-mCherry were expressed in the *E. coli* BL21 system and purified by affinity chromatography followed by FPLC. As a result of the purification, high-purity proteins were obtained with a high yield of about 4 mg or more per 1 liter of the medium. After the purification, the protein sample was separated by SDS-PAGE, stained with Coomassie brilliant blue and then visualized after destaining (FIG. 1b). The predicted molecular weights of the proteins were 33.64 kDa and 32.65 kDa for the C'-GFP and C-mCherry, respectively, which were similar to the molecular weights estimated from the SDS-PAGE result around the 35-kDa marker.

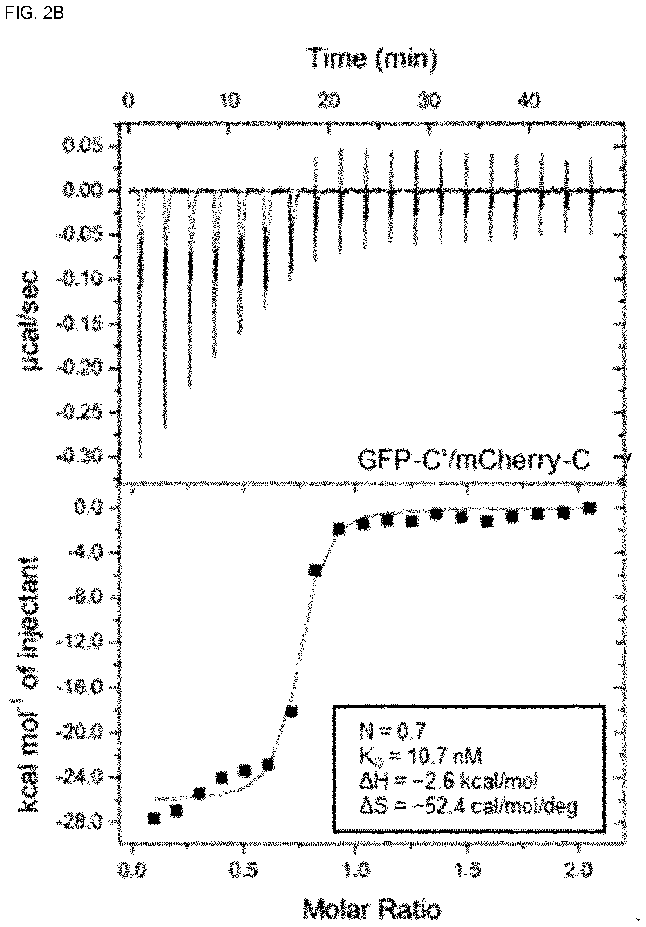

After the purification, native PAGE was performed to confirm whether the coil-fused fluorescent proteins were folded properly. As a result of the native PAGE, it was confirmed that green fluorescence and red fluorescence are observed when C'-GFP and C-mCherry exist alone (FIG. 2a). However, when C'-GFP and C-mCherry were mixed at a ratio of 1:1, yellow fluorescence was observed as the green fluorescence and red fluorescence were combined. This indicates that each fluorescent protein is folded correctly and the fused coil self-assembles in a complementary manner. Binding parameters were determined by investigating the thermodynamics between C'-GFP and C-mCherry by isothermal titration calorimetry (ITC). The parameters measured for C'-GFP and C-mCherry were: N = 0.7, $K_D$ = 10.7 nM, $\Delta H$ = -2.6 kcal/mol and $\Delta S$ = -52.4 cal/mol/deg. The interaction between the two molecules was spontaneous and it was driven by high binding affinity (FIG. 2b). In addition, C'-GFP and C-mCherry interacted at a ratio of 0.7:1, which was lower than the theoretical ratio of 1:1. This may be due to homodimer formation between the coils or an error caused by the difference between the actual concentration and the concentration used to fit the isotherm. In summary, it was confirmed that C'-GFP and C-mCherry, which can be folded accurately and can form a heterodimeric complex by binding with high affinity, were expressed.

**2. Designing of coiled cysteine mutant and evaluation of stability**

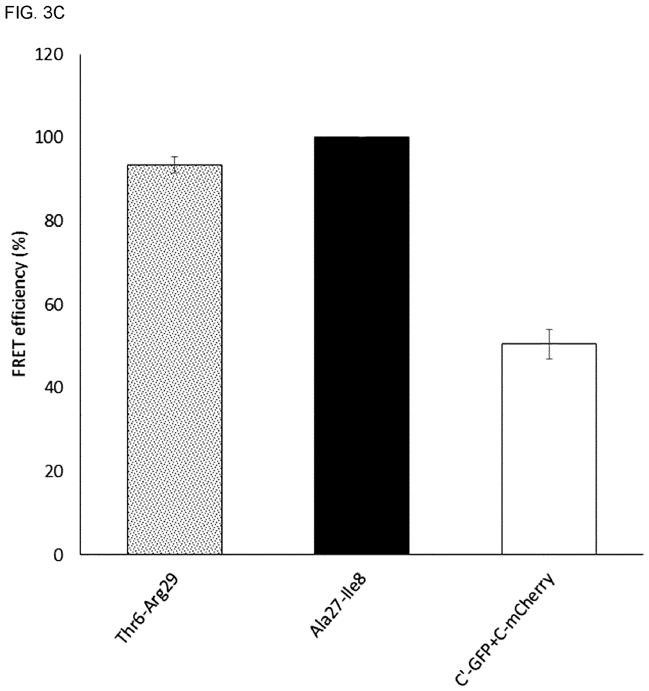

A disulfide bridge formed between two thiol groups of cysteine (Cys) residues in the same or different molecules can serve as an important component for correct protein folding and structural stability. In order to provide improved resistance to dissociation during self-assembly, the inventors of the present disclosure sought to form a disulfide bond coiled coil complex. To this end, they searched for a pair of residues that can form a disulfide bond while minimizing the coiled-coil interaction. C'_Ile8-C_Ala27 (I8C-A27C) (SEQ ID NO 7 + SEQ ID NO 2) and C'_Arg29-C_Thr6 (R29C-T6C) (SEQ ID NO 8 + SEQ ID NO 3) were judged to be the optimal crosslinkage sites where the disulfide bond can be formed while maintaining good electrostatic interaction. In order to investigate the degree of inhibition of coiled-coil interaction by mutation, the interaction residue pairs exhibiting good electrostatic interaction, C'_Glu18-C_Arg16 (E18C-R16C) (SEQ ID NO 9 + SEQ ID NO 4) and C'_Leu1 1-C_Val20 (L11C-V20C) (SEQ ID NO 10 + SEQ ID NO 5), were selected for comparison. As a result of analyzing the coiled-coil interaction of wild-type GFP-C' and wild-type mCherry-C depending on DTT and the presence or absence of mutation (SEQ ID NO 6 + SEQ ID NO 1) (GFP-C' + mCherry-C) by native PAGE, it was confirmed that C'_Ile8-C_Ala27 (I8C-A27C) (SEQ ID NO 7 + SEQ ID NO 2) (Ala27-Ile8) and C'_Arg29-C_Thr6 (R29C-T6C) (SEQ ID NO 8 + SEQ ID NO 3) (Thr6-Arg29) form a more stable complex than C'_Glu18-C_Arg16 (E18C-R16C) (SEQ ID NO 9 + SEQ ID NO 4) (Coiled coil_F1) and C'_Leu1 1-C_Val20 (L11C-V20C) (SEQ ID NO 10 + SEQ ID NO 5) (Coiled coil_F2) (FIG. 3a). In addition, as a result of comparing the relative expression level, the expression level of Ala27-Ile8 and Thr6-Arg29 was 2 times or higher than Coiled coil_F1 and Coiled coil_F2 (FIG. 3b). In addition, as a result of quantitatively analyzing the fluorescence resonance energy transfer efficiency between the GFP and mCherry molecules resulting from the stable coiled-coil interaction between Ala27-Ile8 and Thr6-Arg29 (FRET analysis), it was confirmed that the efficiency was 2 times or higher as compared to wild-type (GFP-C' + mCherry-C) (FIG. 3c).

**3. Preparation of TRAIL trimer-coil and TRAIL hexamer**

**3.1. Cellular expression and purification**

Two proteins (TRAIL trimer-MBD2 (scTRAIL-C) and TRAIL trimer-p66α (scTRAIL-C')) were expressed by culturing transformed *E. coli* and purified by affinity chromatography. More specifically, TRAIL trimer-MBD and TRAIL trimer-p66α were expressed and purified by the following procedures:

Transformed BL21 (DE3) *E. coli* cells were cultured in solid LB medium containing 0.1 mg/mL ampicillin concentration for 12 hours. Then, the cells wherein the insertion of the vector was confirmed were inoculated to 2xYT medium (ampicillin 0.1 mg/mL, the same hereinafter) and cultured at 37 °C for 12 hours. The cells were inoculated again to 2xYT medium diluted to 1:100 and cultured at 30 °C while stirring at 200 rpm. When the absorbance at 600 nm reached 0.4-0.5, IPTG was added to a final concentration of 1.0 mM to induce protein expression and the temperature was lowered to 16-18 °C. After culturing for 20-40 hours, the cells were centrifuged at 4000 g for 10 minutes and the cell pellets were collected and stored at -20 °C.

For purification of proteins, the cells were resuspended by adding 40 mL of a lysis buffer (pH 8.0, 50 mM sodium phosphate, 0.3 M NaCl and 10 mM imidazole) to the pellets. After adding lysozyme to a final concentration of 1 mg/mL, mixing was conducted for 30 minutes while rotating at 4 °C. Then, after sonication for 8 minutes with 10-second intervals, proteins and cell debris were separated by centrifuging at 10,000 g for 30 minutes. The supernatant containing proteins was transferred separately and, after adding 1.5 mL of Ni-NTA agarose beads, mixed by rotating at 4 °C for 30 minutes. After fixing a gravity-flow column, the supernatant mixed with Ni-NTA was flown through the column. Then, in order to wash the beads, a wash buffer (pH 8.0, 50 mM sodium phosphate, 0.3 M NaCl and 20 mM imidazole) was flown sufficiently and the impurities adhered to the beads were removed until the absorbance of the solution at 280 nm reached 0.00. In order to separate the proteins bound to the beads, 500 µL of an elution buffer (50 mM sodium phosphate (pH 8.0), 0.3 M NaCl and 250 mM imidazole) was flown and the eluate was transferred to a microtube. The concentration of the eluate was measured at 280 nm using Biodrop and then stored at 4 °C.

**3.2. Characterization**

The affinity of 1) TRAIL trimer-C (scTRAIL-C) and TRAIL trimer-C' (scTRAIL-C'), 2) TRAIL trimer-C or TRAIL trimer-C' and a death receptor (DR) and 3) TRAIL hexamer (scTRAIL-C + scTRAIL-C') and a death receptor (DR) was measured by binding assay (MicroScale Thermophoresis, MST). DR is a cognate receptor of TRAIL.

Details are as follows:

For sample pretreatment prior to analysis, a death receptor (Biolegend) and Flamma® 648 Sulfo-NHS ester dissolved in DMSO were mixed at a molar ratio of 1:5 and reacted at room temperature for 1 hour. Before sample injection, a Zeba™ spin desalting column (40 K MWCO, 0.5 mL) was centrifuged at 1500 g for 1 minute to remove the storage solution. Then, after filling the column again with 450 µL of a 150 mM sodium carbonate solution, centrifugation was performed again at 1500 g for 1 minute. Afterwards, the 100-150 µL of the reacted DR mixture was centrifuged at 1500 g for 2 minutes to remove a trace amount of DMSO contained in the sample. Then, the sodium carbonate solution was exchanged and the non-labeled and labeled DRs were separated.

Before analyzing the sample using the Monolith NT.115 (Germany) MST instrument, the measurement intensity was adjusted using the labeled DR. After filling the labeled DR of the same concentration in four identical capillaries, measurement was made while adjusting the excitation power. The fluorescence intensity value ranged from 800 to 1000.

The recombinant proteins, i.e., single-molecule TRAIL (MonoTRAIL), TRAIL trimer-C and TRAIL trimer-C, or TRAIL hexamer, were used as ligands. The receptor and the ligand were mixed as follows. First, 10 µL of the death receptor protein was dispensed into 15 racks excluding the first rack. Then, 20 µL of TRAIL trimer-C as the ligand was added to the first rack. Subsequently, 10 µL was taken from the first rack and diluted in the second rack, and 10 µL was taken from the second rack and diluted in the third rack. This procedure was repeated 16 times. Finally, the sample in the 16th rack was diluted $2^{16}$ times. After injecting the samples of different concentrations into 16 different capillaries and fixing to the detection device, measurement was started. The excitation power was increased to twice of the initially set value. The same procedures were performed for TRAIL trimer-C' and TRAIL hexamer.

As a result, it was confirmed that the affinity of the TRAIL hexamer and the death receptor (163 nM) was about 2-5 times higher than that of the TRAIL trimer-C or TRAIL trimer-C' and the death receptor (337 nM and 787 nM, respectively) (FIG. 4).

**3.3. *In vitro* cell assay**

U87-luc glioblastoma cells (PerkinElmer, Akron, Ohio, USA) were treated with a positive control group (R&D TRAIL, R&D systems, Minneapolis, MN, USA), TRAIL trimer-C (I), TRAIL trimer-C' (II) or TRAIL hexamer (I + II) for 4 hours and observed by flow cytometry (FACS).

As a result, whereas the TRAIL trimer-C (I) or TRAIL trimer-C' (II) test group exhibited a death rate lower than 5%, the TRAIL hexamer test group (I + II) showed about 30% of death rate, which was about 4 times than that of the positive control group (R&D TRAIL, 8%) (FIGS. 5a and 5b).

While the exemplary embodiments of the present disclosure were described above, those having ordinary knowledge in the art will be able to modify and change the present disclosure variously through addition, change, deletion, etc. of elements without departing from the scope of the present disclosure and such modifications and changes are also included in the scope of the present disclosure.

**Claims**

1.  A fusion protein comprising:

    (i) a polypeptide 1 comprising an amino acid sequence of SEQ ID NO 1 or an amino acid sequence wherein an amino acid selected from a group consisting of the 6th and 27th amino acids of SEQ ID NO 1 and a combination thereof is substituted with cysteine;
    (ii) a polypeptide 2 comprising an amino acid sequence of SEQ ID NO 6 or an amino acid sequence wherein an amino acid selected from a group consisting of the 8th and 29th amino acids of SEQ ID NO 6 and a combination thereof is substituted with cysteine; and
    (iii) a drug moiety bound to one or more site selected from a group consisting of the N-terminal and C-terminal of the polypeptides 1 and 2,

    wherein the polypeptides 1 and 2 are bound through self-assembly.

2.  The fusion protein according to claim 1, wherein the drug moiety is an antibody, an antigen-binding fragment thereof or a protein.

3.  The fusion protein according to claim 2, wherein the antigen-binding fragment of the antibody is scFv.

4.   The fusion protein according to claim 2, wherein the protein is an albumin-binding peptide (ABP) or TRAIL (tumor necrosis factor-related apoptosis-inducing ligand).

5.  The fusion protein according to claim 4, wherein the protein is TRAIL trimer.

6.  The fusion protein according to claim 1, wherein the drug moiety is bound to the N-terminal or C-terminal of the polypeptide 1 or 2 via a linker.

7.  A nucleic acid molecule encoding the fusion protein according to claim 1.

8.  A vector comprising the nucleic acid molecule according to claim 7.

9.  An expression construct comprising:

    (i) a nucleic acid molecule 1 encoding a polypeptide 1 comprising an amino acid sequence of SEQ ID NO 1 or an amino acid sequence wherein an amino acid selected from a group consisting of the 6th and 27th amino acids of SEQ ID NO 1 and a combination thereof is substituted with cysteine; and
    (ii) a nucleic acid molecule 2 encoding a polypeptide 2 comprising an amino acid sequence of SEQ ID NO 6 or an amino acid sequence wherein an amino acid selected from a group consisting of the 8th and 29th amino acids of SEQ ID NO 6 and a combination thereof is substituted with cysteine,

    wherein the nucleic acid molecules 1 and 2 are expressed by the same or different vectors so that the polypeptides 1 and 2 can be bound through self-assembly.

10. A host cell comprising the vector according to claim 8 or the expression construct according to claim 9.

11. A composition comprising:

i) a polypeptide 1 comprising an amino acid sequence of SEQ ID NO 2 or 3 or a nucleic acid molecule 1 encoding the polypeptide 1 as a self-assembling domain 1; and

ii) a polypeptide 2 containing an amino acid sequence of SEQ ID NO 7 or 8 or a nucleic acid molecule 2 encoding the polypeptide 2 as a self-assembling domain 2.

12. A pharmaceutical composition for preventing or treating cancer, comprising the fusion protein according to claim 1, the nucleic acid molecule according to claim 7, the vector according to claim 8, the expression construct according to claim 9, the host cell according to claim 10 or the composition according to claim 11.

13. A method for treating cancer, comprising a step of administering the fusion protein according to claim 1, the nucleic acid molecule according to claim 7, the vector according to claim 8, the expression construct according to claim 9, the host cell according to claim 10 or the composition according to claim 11 to a subject in need thereof.

14. A kit comprising:

(i) a polypeptide 1 comprising an amino acid sequence wherein an amino acid selected from a group consisting of the 6th and 27th amino acids of SEQ ID NO 1 and a combination thereof is substituted with cysteine or a nucleic acid molecule 1 encoding the same; and

(ii) a polypeptide 2 comprising an amino acid sequence wherein an amino acid selected from a group consisting of the 8th and 29th amino acids of SEQ ID NO 6 and a combination thereof is substituted with cysteine or a nucleic acid molecule 2 encoding the same; and

(iii) a nucleotide sequence encoding a drug moiety bound to a site selected from a group consisting of the N-terminal and C-terminal of the polypeptides 1 and 2 or the 5'- or 3'-end of the nucleic acid molecules 1 and 2.

15. The kit according to claim 14, wherein the drug moiety is TRAIL.

FIG. 1A

FIG. 1B

FIG. 2A

FIG. 2B

GFP-C'/mCherry-C

N = 0.7
$K_D$ = 10.7 nM
$\Delta H$ = −2.6 kcal/mol
$\Delta S$ = −52.4 cal/mol/deg

FIG. 3A

→ w/o DTT

→ w/ DTT

FIG. 3B

FIG. 3C

FIG. 4

FIG. 5A

FIG. 5B

**U87-Luc**

| CTL | Control |
| PC | R&D TRAIL |
| T1 | I scTRAIL-C' |
| T2 | II scTRAIL-C |
| T3 | I+II |

## INTERNATIONAL SEARCH REPORT

| International application No. |
|---|
| **PCT/KR2022/014508** |

| A. | CLASSIFICATION OF SUBJECT MATTER |
|---|---|

**A61K 47/42**(2006.01)i; **A61K 47/64**(2017.01)i; **C07K 14/47**(2006.01)i; **C07K 1/16**(2006.01)i; **C12N 15/70**(2006.01)i; **C07K 14/705**(2006.01)i; **A61K 38/00**(2006.01)i; **A61P 35/00**(2006.01)i

According to International Patent Classification (IPC) or to both national classification and IPC

| B. | FIELDS SEARCHED |
|---|---|

Minimum documentation searched (classification system followed by classification symbols)

A61K 47/42(2006.01); A61K 31/40(2006.01); A61P 35/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: MBD2, p66α, 코일드 코일(coiled coil), 약물 전달(drug delivery), 치료(treatment)

| C. | DOCUMENTS CONSIDERED TO BE RELEVANT |
|---|---|

| Category* | Citation of document, with indication, where appropriate, of the relevant passages | Relevant to claim No. |
|---|---|---|
| X<br>Y<br>A | WALAVALKAR, Ninad M. et al. Unique features of the anti-parallel, heterodimeric coiled-coil interaction between methyl-cytosine binding domain 2 (MBD2) homologues and GATA zinc finger domain containing 2A (GATAD2A/p66α). Journal of Biological Chemistry. 2013, vol. 288, no. 5, pp. 3419-3427.<br>See page 3420; and figure 1. | 9-10<br>1-8<br>11,14-15 |
| Y | UTTERSTROM, Johanna et al. Coiled coil-based therapeutics and drug delivery systems. Advanced Drug Delivery Reviews. 27 December 2020 (online publication date), vol. 170, pp. 26-43.<br>See abstract; and page 38. | 1-8 |
| X | KIM, Min Young et al. Rational discovery of antimetastatic agents targeting the intrinsically disordered region of MBD2. Science Advances. 2019, vol. 5, eaav9810, pp. 1-11.<br>See pages 3 and 5. | 9-10 |
| A | MCFARLANE, Ainsley A. et al. The use of coiled-coil proteins in drug delivery systems. European Journal of Pharmacology. 2009, vol. 625, pp. 101-107.<br>See entire document. | 1-11,14-15 |

| ☑ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **27 December 2022** | **27 December 2022** |

| Name and mailing address of the ISA/KR | Authorized officer |
|---|---|
| **Korean Intellectual Property Office**<br>**Government Complex-Daejeon Building 4, 189 Cheongsa-**<br>**ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
|---|
| **PCT/KR2022/014508** |

**C. DOCUMENTS CONSIDERED TO BE RELEVANT**

| Category* | Citation of document, with indication, where appropriate, of the relevant passages | Relevant to claim No. |
|---|---|---|
| A | US 10610514 B2 (INDUSTRY-UNIVERSITY COOPERATION FOUNDATION HANYANG UNIVERSITY) 07 April 2020 (2020-04-07)<br>See entire document. | 1-11,14-15 |

Form PCT/ISA/210 (second sheet) (July 2022)

**INTERNATIONAL SEARCH REPORT**

| International application No. |
| --- |
| **PCT/KR2022/014508** |

| Box No. I | Nucleotide and/or amino acid sequence(s) (Continuation of item 1.c of the first sheet) |
| --- | --- |

1.  With regard to any nucleotide and/or amino acid sequence disclosed in the international application, the international search was carried out on the basis of a sequence listing:

   a.  ☑ forming part of the international application as filed.

   b.  ☐ furnished subsequent to the international filing date for the purposes of international search (Rule 13*ter*.1(a)),

      ☐ accompanied by a statement to the effect that the sequence listing does not go beyond the disclosure in the international application as filed.

2.  ☐ With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

| International application No. |
| --- |
| **PCT/KR2022/014508** |

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **13**
    because they relate to subject matter not required to be searched by this Authority, namely:

    Claim 13 pertains to a method for treatment of the human body by therapy (PCT Article 17(2)(a)(i) and PCT Rule 39.1(iv)).

2. ☐ Claims Nos.:
    because they relate to parts of the international application that do not comply with the prescribed requirements to such an extent that no meaningful international search can be carried out, specifically:

3. ☑ Claims Nos.: **12-13**
    because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

| | International application No. |
| --- | --- |
| | **PCT/KR2022/014508** |

| Patent document<br>cited in search report | Publication date<br>(day/month/year) | Patent family member(s) | | | Publication date<br>(day/month/year) |
| --- | --- | --- | --- | --- | --- |
| US        10610514      B2 | 07 April 2020 | KR   10-2018-0058343 | | A | 01 June 2018 |
| | | KR   10-2018-0058344 | | A | 01 June 2018 |
| | | KR          10-2022154 | | B1 | 18 September 2019 |
| | | KR          10-2026516 | | B1 | 27 September 2019 |
| | | US        2018-0250266 | | A1 | 06 September 2018 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140073566 A1
- US 6316652 B
- US 6274552 B
- US 6271359 B
- US 6253872 B
- US 6139865 A
- US 6131570 A
- US 6120751 A
- US 6071495 A
- US 6060082 A
- US 6048736 A
- US 6039975 A
- US 6004534 A
- US 5985307 A
- US 5972366 A
- US 5900252 A
- US 5840674 A
- US 5759542 A
- US 5709874 A

**Non-patent literature cited in the description**

- **ZORZI et al.** *Medchemcomm.,* 01 July 2019, vol. 10 (7), 1068-1081
- **SCHEIT.** Nucleotide Analogs. John Wiley, 1980
- **UHLMAN ; PEYMAN.** *Chemical Reviews,* 1990, vol. 90, 543-584
- **MANIATIS et al.** Molecular Cloning, A Laboratory Manual. Cold Spring Harbor Press, 1988
- **AUSUBEL et al.** Current Protocols in Molecular Biology. John, Wiley & Sons, Inc, 1994
- Remington's Pharmaceutical Sciences. Mack Publishing Co, 1990